# EUROPÄISCHE PATENTANMELDUNG

(11) **EP 2 305 111 A1**
(43) Veröffentlichungstag der Anmeldung: **06.04.2011**
(21) Anmeldenummer: 09171972.4
(22) Anmeldetag: 01.10.2009
(51) Int. Cl.: A61B 5/053

(54) **Bioimpedanzmessvorrichtung und -verfahren**

(71) Anmelder: seca ag, 4153 Reinach BL 1 (CH)
(72) Erfinder: Jensen, Björn, 22043, Hamburg (DE)
(74) Vertreter: UEXKÜLL & STOLBERG

(57) **Zusammenfassung**

Die Erfindung betrifft eine Bioimpedanzmessvorrichtung zur Bestimmung von Zusammensetzungsdaten des menschlichen Körpers mit mindestens acht Elektroden, die paarweise jeweils einem der vier Gliedmaßen zugeordnet sind, Messschaltungen mit Stromquellen und Strom- und Spannungsmessschaltungen, die auswählbar mit den Elektroden verbindbar sind, und einer Steuer- und Auswerteeinheit, die dazu vorbereitet ist, nach Maßgabe von mehreren vorbestimmten Messprogrammen jeweils über zwei für das jeweilige Messprogramm spezifische Elektroden einen Wechselstrom aus den Stromquellen in den Körper einzuprägen und mit zwei anderen spezifischen Elektroden an verschiedenen Gliedmaßen mit den Spannungsmessschaltungen die resultierende Spannungen festzustellen und daraus die Impedanz von Körpersegmenten zu bestimmen. Erfindungsgemäß ist die Steuer- und Auswerteeinheit weiter dazu vorbereitet, in einer Vorabmessung durch Einprägen von Strom über zwei Elektroden und Messung von resultierendem Strom durch die einprägenden Elektroden und/oder Spannungen an den übrigen Elektroden festzustellen, welche Elektroden Kontakt haben, und nach Maßgabe der festgestellten Konfiguration von Elektroden mit Kontakt dazu solche passenden Messprogramme auszuwählen, bei denen nur Elektroden zur Stromeinprägung und Spannungsmessung vorgesehen sind, die in der festgestellten Konfiguration enthalten sind.

## Beschreibung

Die vorliegende Erfindung betrifft eine Bioimpedanzmessvorrichtung zur Bestimmung von Zusammensetzungsdaten des menschlichen Körpers und ein entsprechendes Messverfahren.

Die Leitfähigkeit des menschlichen Körpers wird stark durch den Wasseranteil beeinflusst. Da die fettfreien Anteile des Körpers wie Muskeln und die Körperflüssigkeiten einen Großteil des körpereigenen Wassers beinhalten, während Fettgewebe einen nur relativ geringen Wasseranteil besitzt, kann durch die Bestimmung der Leitfähigkeit des Körpers oder eines Köpersegments (oder umgekehrt des Widerstands oder der Impedanz des Körpers oder des Körpersegments) ein Rückschluss auf den relativen Anteil von Fett gezogen werden, zumindest wenn weitere Daten wie die Größe und das Gewicht der Person berücksichtigt werden.

Ein Verfahren und eine Vorrichtung zur Bioimpedanzanalyse sind z.B. in WO 97/01303 beschrieben. Die beschriebene Vorrichtung weist acht Elektroden auf, nämlich vier Fußelektroden, jeweils zwei zum Kontaktieren eines Fußes, und vier Handelektroden, jeweils zwei zur Kontaktierung an einer Hand der Person. Dann wird ein Wechselstrom über jeweils zwei, an unterschiedlichen Gliedmaßen befindliche Elektroden eingeprägt und an zwei, ebenfalls an unterschiedlichen Gliedmaßen anliegenden Elektroden die Spannung gemessen. Durch Übergang zu anderen Paaren von stromeinprägenden Elektroden und spannungserfassenden Elektroden können sukzessive verschiedene Körpersegemente untersucht werden. Ferner kann bei Stromeinspeisung in eine Hand und einen Fuß und Spannungsmessung an derselben Hand und an demselben Fuß eine ganze Körperseite gemessen werden.

Wenn jeweils alle Elektroden an Händen und Füßen Kontakt haben, steht also eine Mehrzahl von Messprogrammen oder Konfigurationen zur Verfügung, welche Elektroden als stromeinprägende und welche als spannungsmessende fungieren, wobei die Konfiguration von stromeinprägenden Elektroden und spannungsmessenden Elektroden bestimmt, welches Körpersegment oder ob der Gesamtkörper gemessen wird. Wenn an jedem der Gliedmaßen zwei Elektroden Kontakt haben, steht also eine Mehrzahl von möglichen Messprogrammen zur Verfügung. Andererseits kann es bei der Verwendung von Klebelektroden vorkommen, dass auf Messprogramme verzichtet werden soll, wenn dafür weniger Elektroden verwendet werden. Außerdem gibt es Situationen, in denen die zu untersuchende Person nicht mit allen Gliedmaßen mit Elektroden in Kontakt kommen kann, z.B. wenn ein Gliedmaß amputiert ist oder durch Bandagen oder Gipsverband verdeckt ist. In dieser Situation wird die zu untersuchende Person die Messvorrichtung mit den ihr möglichen Gliedmaßen kontaktieren. Dann muß die zu untersuchende Person selbst oder eine Bedienungsperson das Messprogramm auswählen, das ausgeführt werden soll. Wie erwähnt können dies je nach Anzahl der Kontaktpunkte ein oder mehrere mögliche Messprogramme sein.

In EP 2 042 843 A1 wurde vorgeschlagen, eine Bioimpedanzmessvorrichtung so auszubilden, dass die Bioimpedanzmesseinheit eingeschaltet wird, wenn festgestellt wird, dass wenigstens zwei der Elektroden der Vorrichtung in Kontakt mit der zu untersuchenden Person sind, oder andernfalls deaktiviert wird.

Es ist Aufgabe der vorliegenden Erfindung, eine Bioimpedanzmessvorrichtung mit mindestens acht Elektroden zum paarweisen Kontakt an den vier Gliedmaßen einer zu untersuchenden Person so auszubilden, dass sie für die zu untersuchende Person und/oder das Bedienungspersonal möglichst effektiv und einfach zu handhaben ist, und ein entsprechendes Verfahren anzugeben.

Zur Lösung dieser Aufgabe wird eine Bioimpedanzmessvorrichtung mit den Merkmalen des Patentanspruchs 1 und ein Bioimpedanzmessverfahren mit den Merkmalen des Patentanspruchs 6 angegeben. Vorteilhafte Ausführungsformen sind in den Unteransprüchen aufgeführt.

Die erfindungsgemäße Bioimpedanzmessvorrichtung ist durch ihre Steuer- und Auswerteeinheit dazu vorbereitet, eine Vorabmessung durchzuführen und festzustellen, welche der Elektroden Kontakt am Körper haben. Die Steuer- und Auswahleinheit ist weiter dazu vorbereitet, nach Ermittlung der Konfiguration von Elektroden mit Kontakt aus einer Mehrzahl von vorab gespeicherten Messprogrammen diejenigen auszuwählen, die zu der festgestellten Konfiguration von Elektroden mit Kontakt passen, d.h. solche Messprogramme, bei denen nur Elektroden zur Stromeinprägung und Spannungsmessung vorgesehen sind, die in der festgestellten Konfiguration enthalten sind. Der Kontakt von Elektroden wird festgestellt, indem Strom über zwei Elektroden eingeprägt und der resultierende Strom durch die einprägenden Elektroden und/oder Spannungen an den übrigen Elektroden erfasst werden. Der Kontakt wird für eine Elektrode festgestellt, wenn die gemessenen Werte innerhalb vorgegebener Grenzwerte liegen. Der vorab eingeprägte Strom kann ein Wechselstrom oder ein Gleichstrom sein.

Die Steuer- und Auswerteeinheit kann so ausgestaltet sein, dass die Vorabmessung von dem Benutzer gestartet werden muss oder automatisch startet, sobald für wenigstens zwei Elektroden Kontakt festgestellt wird.

Die Erfindung wird im Folgenden an Hand von Ausführungsbeispielen in den Figuren erläutert, in denen:
Figur 1 ein schematisches Schaltbild zur Durchführung eines ersten Messprogramms zur Messung der Impedanz einer Körperseite zeigt,
Figur 2 ein schematisches Schaltbild zu einem zweiten Messprogramm zur Messung der Impedanz eines Armes zeigt,
Figur 3 ein schematisches Schaltbild zu einem dritten Messprogramm zeigt, mit dem die Impedanz eines Beines messbar ist, und
Figur 4 ein schematisches Schaltbild zu einem vierten Messprogramm zeigt, mit dem die Impedanz des Torsos gemessen wird.

Eine typische Bioimpedanzmessvorrichtung weist z.B. eine Standplattform auf, auf die die zu messende Person sich mit beiden Füßen stellt. Auf der Standplattform sind für jeden Fuß zwei Elektroden ausgebildet, z.B. eine im Bereich der Ferse und eine im Bereich des Vorderfußes. Ferner sind zwei Handgriffe vorhanden, die der Benutzer in bestimmter Weise ergreift und damit jede Hand in Kontakt mit zwei Elektroden bringt, z.B. jeweils eine am Daumen und eine an der Handfläche.

Erfindungsgemäß ist die Steuer- und Auswerteeinheit der Bioimpedanzmessvorrichtung dazu vorbereitet, zunächst eine Vorabmessung durchzuführen, mit der ermittelt wird, welche Elektroden elektrischen Kontakt zu der zu messenden Person haben. Dazu können z.B. sukzessive jeweils zwei Elektroden an verschiedenen Gliedmaßen ausgewählt und über sie ein Strom (dabei kann es sich in der Vorabmessung um einen Wechselstrom oder Gleichstrom handeln) eingeprägt werden, der über die Elektroden fließende Strom wird gemessen, und es wird festgestellt, dass die beiden Elektroden Kontakt haben, wenn der gemessene Strom innerhalb vorgegebener Stromgrenzwerte liegt. Dies kann für weitere Elektrodenpaare sukzessive solange durchgeführt werden, bis für alle Elektroden feststeht, ob sie Kontakt haben oder nicht. Alternativ kann ergänzend zu der Einprägung von Strom über zwei Elektroden mit zwei anderen Elektroden der resultierende Spannungsabfall gemessen werden und für diese anderen Elektroden festgestellt werden, dass sie Kontakt haben, wenn die gemessenen Spannungsabfälle innerhalb vorgegebener Spannungsgrenzwerte liegen.

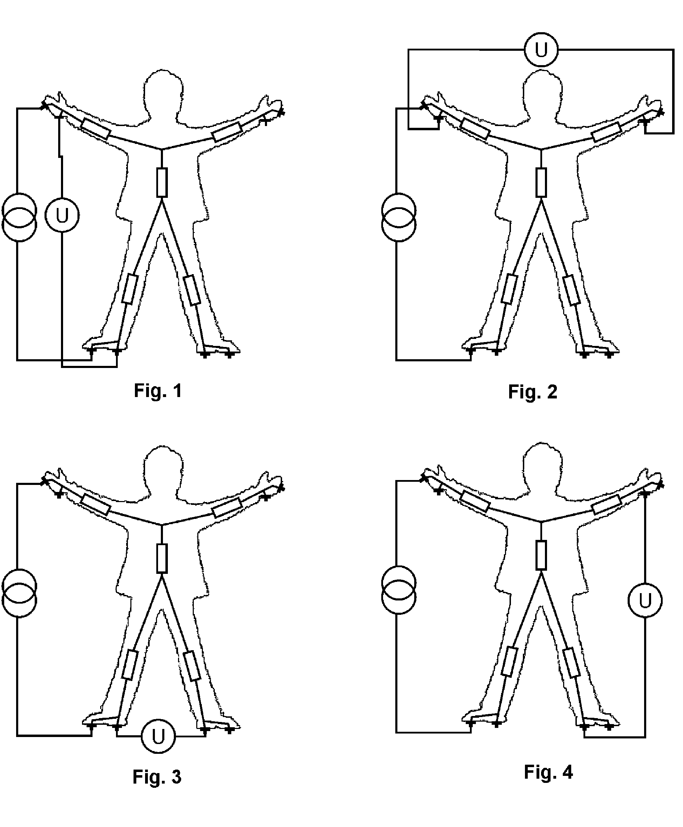

Wenn z.B. festgestellt worden ist, dass acht Elektroden, je zwei an jedem der Gliedmaßen, Kontakt haben, stehen eine Reihe von möglichen Messprogrammen zur Verfügung, z.B. das aus Figur 1, bei dem Wechselstrom über Elektroden an einer Hand und an einem Fuß der gleichen Körperseite eingeprägt wird und die resultierende Spannung an derselben Hand und demselben Fuß mit der jeweils anderen Elektrode gemessen wird; dieses Messprogramm ist sensitiv für die Impedanz einer ganzen Körperseite, d.h. ist ein Maß für die Ganzkörperimpedanz.

Bei dem in Figur 2 dargestellten Messprogramm wird der Wechselstrom wiederum über eine Elektrode an einer Hand und eine Elektrode an einem Fuß der gleichen Körperseite eingespeist und die Spannung an der anderen Elektrode der einen Hand und der anderen Hand gemessen. Diese Impedanzmessung ist sensitiv für die Impedanz des stromdurchflossenen Armes.

Bei dem in Figur 3 dargestellten Messprogramm wird der Wechselstrom in gleicher Weise wie zuvor eingeprägt und die Spannung über eine Elektrode an dem einen und eine Elektrode an dem anderen Fuß gemessen. Diese Impedanzmessung ist sensitiv für die Impedanz des stromdurchflossenen Beines.

Bei dem in Figur 4 dargestellten Messprogramm wird der Wechselstrom in gleicher Weise wie zuvor eingeprägt und die Spannung über eine Elektrode an der Hand und am Fuß an der gegenüberliegenden Körperseite gemessen. Diese Impedanzmessung ist sensitiv für die Impedanz des Torsos.

Wird in der Vorabmessung festgestellt, dass acht Messelektroden Kontakt haben, je zwei für jedes der Gliedmaßen, so stehen alle Messprogramme wie in Figuren 1-4 gezeigt (und deren Permutationen, d.h. Vertauschungen über die Gliedmaßen) zur Verfügung und werden nach Ausführung der Vorabmessung ausgewählt und als ausführbar bereitgestellt, z.B. als Alternativen auf einer Anzeige dargestellt, aus der der Benutzer dann auswählen kann oder es werden alle Messprogramme ausgeführt.

Wird in der Vorabmessung festgestellt, dass z.B. die Elektroden einer Hand keinen Kontakt haben, wählt die Steuer- und Auswerteeinheit nur solche Messprogramme aus, die zu der Konfiguration der Elektroden mit Kontakt passen. Diese wären aus den Beispielen in Figuren 1-4 die Messprogramme aus Figur 1 und Figur 3. Diese Messprogramme werden dann ausgewählt und als ausführbar bereitgestellt.

Wird in der Vorabmessung festgestellt, dass z.B. nur die Elektroden an einer Hand und einem Fuß an der gleichen Körperseite Kontakt haben, so wird das allein dazu passende Messprogramm aus Figur 1 ausgewählt und als ausführbar bereitgestellt.

## Patentansprüche

1. Bioimpedanzmessvorrichtung zur Bestimmung von Zusammensetzungsdaten des menschlichen Körpers mit mindestens acht Elektroden, die paarweise jeweils einem der vier Gliedmaßen zugeordnet sind, Messschaltungen mit Stromquellen und Strom- und Spannungsmessschaltungen, die auswählbar mit den Elektroden verbindbar sind, und einer Steuer- und Auswerteeinheit, die dazu vorbereitet ist, nach Maßgabe von mehreren vorbestimmten Messprogrammen jeweils über zwei für das jeweilige Messprogramm spezifische Elektroden einen Wechselstrom aus den Stromquellen in den Körper einzuprägen und mit zwei anderen spezifischen Elektroden an verschiedenen Gliedmaßen mit den Spannungsmessschaltungen die resultierende Spannungen festzustellen und daraus die Impedanz von Körpersegmenten zu bestimmen, **dadurch gekennzeichnet, dass** die Steuer- und Auswerteeinheit weiter dazu vorbereitet ist, in einer Vorabmessung durch Einprägen von Strom über zwei Elektroden und Messung von resultierendem Strom durch die einprägenden Elektroden und/oder Spannungen an den übrigen Elektroden festzustellen, welche Elektroden Kontakt haben, und nach Maßgabe der festgestellten Konfiguration von Elektroden mit Kontakt dazu solche passenden Messprogramme, bei denen nur Elektroden zur Stromeinprägung und Spannungsmessung vorgesehen sind, die in der festgestellten Konfiguration enthalten sind, auszuwählen.

2. Bioimpedanzmessvorrichtung nach Anspruch 1, **dadurch gekennzeichnet, dass** die Steuer- und Auswerteeinheit weiter dazu vorbereitet ist, in der Vorabmessung eine Einprägung von Strom über zwei Elektroden durchzuführen und den Strom durch die zwei Elektroden zu erfassen und festzustellen, dass diese Elektroden Kontakt haben, wenn der erfaßte Strom innerhalb vorgegebener Stromgrenzwerte liegt.

3. Bioimpedanzmessvorrichtung nach Anspruch 2, **dadurch gekennzeichnet, dass** die Steuer- und Auswerteeinheit weiter dazu vorbereitet ist, bei der Vorabmessung Strom über mehr als zwei Elektroden einzuprägen, indem der Strom sukzessive über verschiedene Kombinationen von jeweils zwei Elektronen eingeprägt, jeweils der Strom erfaßt und festgestellt wird, dass diese Elektroden Kontakt haben, wenn der erfaßte Strom innerhalb der vorgegebenen Stromgrenzwerte liegt.

4. Bioimpedanzmessvorrichtung nach Anspruch 1, **dadurch gekennzeichnet, dass** die Steuer- und Auswerteeinheit weiter dazu vorbereitet ist, bei der Vorabmessung Strom über mindestens zwei Elektroden einzuprägen und über mindestens zwei andere Elektroden die über den betroffenen Körperteil abfallende Spannung zu erfassen, wobei als stromeinprägende Elektroden solche ausgewählt werden, bei denen bei Stromeinprägung und Messung der durch diese Elektroden fließenden Ströme diese innerhalb der vorgegebenen Grenzwerte lagen, wobei die erfaßten abfallenden Spannungen überprüft werden und Kontakt für die den Spannungsabfall erfassenden Elektroden festgestellt wird, wenn die erfaßten abfallenden Spannungen innerhalb vorgegebener Spannungsgrenzwerte liegen.

5. Bioimpedanzmessvorrichtung nach einem der vorhergehenden Ansprüche, **dadurch gekennzeichnet, dass** die Steuer- und Auswerteeinheit weiter dazu vorbereitet ist, nach einer Vorabmessung, falls mehrere Messprogramme zu der ermittelten Konfiguration von Elektroden mit Kontakt passen, die ausgewählten Messprogramme auf einer Anzeige anzuzeigen.

6. Bioimpedanzmessverfahren zur Bestimmung von Zusammensetzungsdaten des menschlichen Körpers, bei dem jeweils zwei Elektroden jedem der Gliedmaßen des Körpers zugeordnet sind, nach Maßgabe von mehreren vorbestimmten Messprogrammen jeweils über zwei für das jeweilige Messprogramm spezifische Elektroden ein Wechselstrom in den Körper eingeprägt und mit zwei andern spezifischen Elektroden an verschiedenen Gliedmaßen die resultierende Spannungen erfasst und daraus die Impedanz desjenigen Körpersegments bestimmt wird, für das das Messprogramm spezifisch ist, **dadurch gekennzeichnet, dass** vor dem Start eines Messprogramms eine Vorabmessung durchgeführt wird, indem sukzessive über verschiedene Elektrodenpaare ein Strom eingeprägt und der resultierende Strom durch die einprägenden Elektroden und/oder Spannungen an den übrigen Elektroden gemessen werden, um für Elektroden, deren Messwerte innerhalb vorgegebener Grenzwerte liegen, festzustellen, dass sie Kontakt haben, und dass nach Maßgabe der festgestellten Konfiguration von Elektroden mit Kontakt zu dieser Konfiguration passende Messprogramme ausgewählt werden, bei denen nur Elektroden zur Stromeinprägung und Spannungsmessung vorgesehen sind, die in der festgestellten Konfiguration enthalten sind.

## Geänderte Patentansprüche

### Geänderte Patentansprüche gemäss Regel 137(2) EPÜ.

**1.** Bioimpedanzmessvorrichtung zur Bestimmung von Zusammensetzungsdaten des menschlichen Körpers mit mindestens acht Elektroden, die paarweise jeweils einem der vier Gliedmaßen zugeordnet sind, Messschaltungen mit Stromquellen und Strom- und Spannungsmessschaltungen, die auswählbar mit den Elektroden verbindbar sind, und einer Steuer- und Auswerteeinheit, die dazu vorbereitet ist, nach Maßgabe von mehreren vorbestimmten Messprogrammen jeweils über zwei für das jeweilige Messprogramm spezifische Elektroden einen Wechselstrom aus den Stromquellen in den Körper einzuprägen und mit zwei anderen spezifischen Elektroden an verschiedenen Gliedmaßen mit den Spannungsmessschaltungen die resultierende Spannungen festzustellen und daraus die Impedanz von Körpersegmenten zu bestimmen, wobei die Steuer- und Auswerteeinheit weiter dazu vorbereitet ist, in einer Vorabmessung durch Einprägen von Strom über zwei Elektroden und Messung von resultierendem Strom durch die einprägenden Elektroden und/oder Spannungen an den übrigen Elektroden festzustellen, welche Elektroden Kontakt haben, und nach Maßgabe der festgestellten Konfiguration von Elektroden mit Kontakt dazu solche passenden Messprogramme, bei denen nur Elektroden zur Stromeinprägung und Spannungsmessung vorgesehen sind, die in der festgestellten Konfiguration enthalten sind, auszuwählen, **dadurch gekennzeichnet, dass** die Steuer- und Auswerteeinheit weiter dazu vorbereitet ist, wenn in der Vorabmessung festgestellt wird, dass acht Elektroden Kontakt haben, je zwei für jedes Gliedmaß, damit ausführbaren Messprogramme zur Messung der Impedanz jeder Körperseite, jedes Arms, jedes Beins und des Torsos auszuführen.

**2.** Bioimpedanzmessvorrichtung nach Anspruch 1, **dadurch gekennzeichnet, dass** die Steuer- und Auswerteeinheit weiter dazu vorbereitet ist, in der Vorabmessung eine Einprägung von Strom über zwei Elektroden durchzuführen und den Strom durch die zwei Elektroden zu erfassen und festzustellen, dass diese Elektroden Kontakt haben, wenn der erfaßte Strom innerhalb vorgegebener Stromgrenzwerte liegt.

**3.** Bioimpedanzmessvorrichtung nach Anspruch 2, **dadurch gekennzeichnet, dass** die Steuer- und Auswerteeinheit weiter dazu vorbereitet ist, bei der Vorabmessung Strom über mehr als zwei Elektroden einzuprägen, indem der Strom sukzessive über verschiedene Kombinationen von jeweils zwei Elektronen eingeprägt, jeweils der Strom erfaßt und festgestellt wird, dass diese Elektroden Kontakt haben, wenn der erfaßte Strom innerhalb der vorgegebenen Stromgrenzwerte liegt.

**4.** Bioimpedanzmessvorrichtung nach Anspruch 1, **dadurch gekennzeichnet, dass** die Steuer- und Auswerteeinheit weiter dazu vorbereitet ist, bei der Vorabmessung Strom über mindestens zwei Elektroden einzuprägen und über mindestens zwei andere Elektroden die über den betroffenen Körperteil abfallende Spannung zu erfassen, wobei als stromeinprägende Elektroden solche ausgewählt werden, bei denen bei Stromeinprägung und Messung der durch diese Elektroden fließenden Ströme diese innerhalb der vorgegebenen Grenzwerte lagen, wobei die erfaßten abfallenden Spannungen überprüft werden und Kontakt für die den Spannungsabfall erfassenden Elektroden festgestellt wird, wenn die erfaßten abfallenden Spannungen innerhalb vorgegebener Spannungsgrenzwerte liegen.

**5.** Bioimpedanzmessvorrichtung nach einem der vorhergehenden Ansprüche, **dadurch gekennzeichnet, dass** die Steuer- und Auswerteeinheit weiter dazu vorbereitet ist, nach einer Vorabmessung, falls mehrere Messprogramme zu der ermittelten Konfiguration von Elektroden mit Kontakt passen, die ausgewählten Messprogramme auf einer Anzeige anzuzeigen.

**6.** Bioimpedanzmessverfahren zur Bestimmung von Zusammensetzungsdaten des menschlichen Körpers, bei dem jeweils zwei Elektroden jedem der Gliedmaßen des Körpers zugeordnet sind, nach Maßgabe von mehreren vorbestimmten Messprogrammen jeweils über zwei für das jeweilige Messprogramm spezifische Elektroden ein Wechselstrom in den Körper eingeprägt und mit zwei andern spezifischen Elektroden an verschiedenen Gliedmaßen die resultierende Spannungen erfasst und daraus die Impedanz desjenigen Körpersegments bestimmt wird, für das das Messprogramm spezifisch ist, wobei vor dem Start eines Messprogramms eine Vorabmessung durchgeführt wird, indem sukzessive über verschiedene Elektrodenpaare ein Strom eingeprägt und der resultierende Strom durch die einprägenden Elektroden und/oder Spannungen an den übrigen Elektroden gemessen werden, um für Elektroden, deren Messwerte innerhalb vorgegebener Grenzwerte liegen, festzustellen, dass sie Kontakt haben, und dass nach Maßgabe der festgestellten Konfiguration von Elektroden mit Kontakt zu dieser Konfiguration passende Messprogramme ausgewählt werden, bei denen nur Elektroden zur Stromeinprägung und Spannungsmessung vorgesehen sind, die in der festgestellten Konfiguration enthalten sind, **dadurch gekennzeichnet, dass** die Steuer- und Auswerteeinheit weiter dazu vorbereitet ist, wenn in der Vorabmessung festgestellt wird, dass acht Elektroden Kontakt haben, je zwei für jedes Gliedmaß, damit ausführbare Messprogramme zur Messung der Impedanz jeder Körperseite, jedes Arms, jedes Beins und des Torsos auszuführen.
